## Europäisches Patentamt
## European Patent Office
## Office européen des brevets

(19)

(11) Numéro de publication: **0 217 687**
**B1**

(12) **FASCICULE DE BREVET EUROPEEN**

(45) Date de publication du fascicule du brevet: **16.01.91**

(51) Int. Cl.⁵: **C 12 P 7/16,** C 12 P 7/28, C 12 N 1/22

(21) Numéro de dépôt: **86401722.3**

(22) Date de dépôt: **31.07.86**

(54) **Procédé de production d'acétone et de butanol a partir de composés lignocellulosiques.**

(30) Priorité: **26.08.85 FR 8512827**

(43) Date de publication de la demande:
**08.04.87 Bulletin 87/15**

(45) Mention de la délivrance du brevet:
**16.01.91 Bulletin 91/03**

(84) Etats contractants désignés:
**BE DE FR GB IT NL**

(56) Documents cités:
**DE-C- 920 724**
**EG-A- 376 698**
**FR-A- 541 535**
**FR-A- 889 580**
**FR-A- 919 525**
**FR-A-2 460 331**
**FR-A-2 490 677**
**GB-A- 222 549**
**US-A-2 203 360**
**US-A-2 386 374**
**US-A-2 421 985**
**US-A-2 431 163**
**US-A-2 444 823**

(73) Titulaire: **INSTITUT FRANCAIS DU PETROLE**
**4, Avenue de Bois-Préau**
**F-92502 Rueil-Malmaison (FR)**

(72) Inventeur: **Marchal, Rémy**
**70, rue des Cormiers**
**F-78400 Chatou (FR)**
Inventeur: **Ropars, Marcel**
**Résidence la Hunière 137, Chemin de la Hunière**
**F-91120 Palaiseau (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

Depuis Pasteur, on sait que la production conjointe d'acétone et de butanol est réalisable par voie fermentaire grâce à la bactérie anaérobie: *Clostridium acetobutylicum*. L'utilisation de ce microorganisme capable de transformer des substrats aussi variés que le saccharose ou l'amidon a permis dans le passé le développement de procédés industriels sur mélasses de betteraves ou farine de maïs. De nos jours, l'exploitation de ces procédés est pratiquement abandonnée en raison de la concurrence de la voie pétrochimique. Depuis quelques années, cependant, l'apparition de nouvelles technologies comme l'utilisation des enzymes a redonné à cette fermentation un regain d'intérêt en rendant réaliste l'utilisation de nouveaux substrats tels que les résidus lignocellulosiques.

La présente invention a donc pour objet la conversion des composés lignocellulosiques tels que pailles de blé, tiges de maïs, copeaux de bois en acétone et butanol par hydrolyse enzymatique et fermentation acétonobutylique. L'hydrolyse enzymatique effectuée par exemple avec les enzymes produites par *Trichoderma reesei*, *Trichoderma viride*, *Trichoderma harzianum*, *Sporotrichum pulverulentum*, conduit respectivement au glucose qui dérive de la cellulose et aux xylose, arabinose, acide glucuronique, qui dérivent des hémicelluloses, tandis que la fermentation acétonobutylique transforme ce mélange d'hexoses et de pentoses en mélange acétone-butanol.

Pour améliorer son accessibilité aux enzymes fongiques, la biomasse lignocellulosique subit avantageusement un prétraitement, par exemple, une hydrolyse acide, un prétraitement dit d'explosion à la vapeur d'eau ou de préférence le traitement décrit dans la demande de brevet français 85 06 027 du 18 avril 1985 qui met en oeuvre une explosion à la vapeur d'eau en présence d'acide. Ce dernier traitement comprend un chauffage du substrat lignocellulosique en présence d'eau et d'acide sous pression superatmosphérique à une température d'au moins 150°C, suivi d'une détente de pression, permettant l'explosion des particules de substrat, le pH étant de préférence de 1 à 4. L'acidité peut être conférée, par exemple, par l'acide sulfurique, l'acide phosphorique, le sulfate acide de sodium, le sulfate acide de potassium ou le sulfate acide d'ammonium. Le traitement acide précité produit une cellulose d'accessibilité améliorée aux cellulases, et d'autre part un mélange de pentoses (xylose, arabinose) résultant de l'hydrolyse directe et quasi totale des hémicelluloses dans les conditions adoptées. Contrairement au prétraitement à la soude, il ne nécessite pas obligatoirement un lavage de la biomasse prétraitée et il conduit à d'excellents rendements d'hydrolyse. Les prétraitements acides ou d'explosion à la vapeur présentent en revanche l'inconvénient de produire des inhibiteurs de fermentation, en particulier de la fermentation acétonobutylique, dérivant peut-être de la dégradation de la lignine ou de certains sucres durant le prétraitement. Pour lever cette inhibition, certains auteurs comme YU et *al* (YU E.K.C., DESCHATELETS L., SADDLER J.N. (1984) Biotechnology letters, *6*, 327—332) ont eu recours à l'extraction à l'eau du substrat prétraité. Ce lavage présente cependant l'inconvénient, comme dans le cas du prétraitement à la soude, d'entraîner, en même temps que les inhibiteurs, une fraction non négligeable des pentoses ce qui entraîne finalement une baisse notable du rendement global de la transformation. D'autres auteurs comme MADDOX et MURRAY (MADDOX I.S. and MURRAY A.E. (1983) Biotechnology letters *5*, 175—178) ont préconisé un entraînement à la vapeur suivi d'un traitement au charbon actif ou bien un double passage sur résines anionique ou cationique. L'efficacité réelle de ces prétraitements reste toutefois limitée puisque la production de butanol ne dépasse pas 5,8 g.1—$^1$.

Une autre technique connue de détoxification (US 2 203 360, 2 421 985 et 2 431 163) consiste à traiter le produit d'hydrolyse acide par la chaux éventuellement additionnée d'un composé de fer ou d'aluminium. Le traitement a lieu à température ordinaire ou faiblement élevée (20 à 40°C). Il se forme un précipité que l'on sépare par filtration ou décantation; la liqueur claire est ensuite soumise à une fermentation.

Le procédé de l'invention est défini comme suit: procédé de production d'acétone et de butanol, dans lequel une matière lignocellulosique est soumise dans l'ordre, à un prétraitement par la vapeur d'eau et/ou par un acide, à une hydrolyse enzymatique par une cellulase et à une fermentation acétonobutylique du produit d'hydrolyse enzymatique, caractérisé en ce qu'après l'hydrolyse enzymatique et avant la fermentation le milieu est chauffé à au moins 50°C en présence d'ions calcium, magnésium et/ou aluminium, et en ce que le produit résultant de ce chauffage, y compris au moins une partie de la fraction insoluble, est soumis à la fermentation acétonobutylique.

La technique de détoxification de l'hydrolysat utilisée par le demandeur dans la présente invention consiste à chauffer le milieu fermentaire en présence d'ion $Ca^{++}$, $Mg^{++}$ ou $Al^{+++}$. Ce préchauffage peut avantageusement consister en la stérilisation du milieu qui précède usuellement la fermentation. On préfère les ions $Ca^{++}$ et $Mg^{++}$. Le pH est avantageusement de 3 à 11, de préférence 6 à 8.

Les ions précités peuvent être ajoutés sous forme de composés quelconques, par exemple oxydes, hydroxydes ou sels, à la paille (ou autre substrat) prétraitée et ou à l'hydrolysat. Comme la stérilisation qui suit habituellement l'hydrolyse et précède la fermentation acétonobutylique a pour effet d'entraîner une baisse de pH de 1,5 unité environ, il peut être avantageux d'ajuster le pH du produit d'hydrolyse à une valeur telle, par exemple environ 8, que le milieu stérile obtenu soit adapté directement à la fermentation acétonobutylique (pH de début de fermentation entre 6 et 7 environ). La fermentation peut être conduite de manière connue à pH contrôlé (le pH de cette fermentation se situe habituellement entre 4 et 8) avec une régulation basique par exemple par addition d'ammoniaque pour éviter une acidification du milieu trop importante durant la phase de production des acides acétique et butyrique.

Les ions $Ca^{++}$, $Mg^{++}$ ou $Al^{+++}$ peuvent être introduits également sous forme de carbonate, de nitrate, de chlorure, d'acétate ou d'autre sel avec une neutralisation de l'hydrolysat par une base soluble telle la soude, la potasse ou l'ammoniaque, cette dernière servant par la même occasion de source d'azote pour le microorganisme. Avec un carbonate, le contrôle du pH est superflu puisque les ions carbonates exercent un rôle tampon qui s'oppose à l'autoacidification. Les ions peuvent être introduits en proportion de, par exemple, 0,3 à 10 $g.1^{-1}$. Le chauffage est effectué à une température supérieure à 50°C. La durée du chauffage ou de la stérilisation peut être variable, par exemple d'une minute à 10 heures, une durée de 10 à 60 minutes étant préférée. Au terme du chauffage ou de la stérilisation, un précipité est présent; au moins une partie de ce précipité est laissée sans dans le milieu.

En résumé, le procédé qui fait l'objet de la présente invention comprend les étapes préférées suivantes:

1) La matière première lignocellulosique subit un prétraitement par la vapeur d'eau, ledit prétraitement étant de préférence un traitement "d'explosion" en milieu acide.

2) La matière première prétraitée est ensuite hydrolysée de manière connue par une composition enzymatique de cellulases produite par exemple par le champignon *Trichoderma reesei*.

3) L'hydrolysat est chauffé à au moins 50°C en présence d'ions calcium, magnésium et/ou aluminium.

4) On effectue de manière connue la fermentation acétonobutylique de l'hydrolysat obtenu après ce chauffage, en y laissant au moins une partie de la fraction insoluble qu'il renferme, de préférence la totalité de cette fraction.

L'hydrolysat peut être amené du pH d'hydrolyse à un pH légèrement alcalin par addition de chaux, ou de magnésie. Alternativement, on peut ajuster le pH au moyen d'un composé basique tel que la potasse, la soude, ou l'ammoniaque et ajouter à l'hydrolysat un sel de calcium ou de magnésium ou d'aluminium.

Dans une variante de l'invention, on ajoute tout ou partie des ions Ca, Mg et Al, par exemple la chaux, la magnésie ou le sel de calcium, de magnésium ou d'aluminium, avant l'étape d'hydrolyse enzymatique.

Les exemples qui suivent illustrent le protocole d'hydrolyse et fermentation de paille de blé prétraitée à la vapeur. Le protocole décrit n'est pas lié à l'utilisation stricte de paille de blé mais convient également pour la transformation de tout autre substrat lignocellulosique tel que des tiges de maïs ou des copeaux de bois De même, l'hydrolyse enzymatique n'est pas liée spécifiquement à l'utilisation du complexe cellulasique produit par *Trichoderma reesei*. L'utilisation qui en a été faite repose avant tout sur les caractéristiques spécifiques du mélange d'enzymes produits, riches en cellulase, hémicellulase et cellobiase.

Exemple 1: traitement avec $Ca^{++}$

1) Prétraitement à la vapeur et hydrolyse enzymatique

Le prétraitement à la vapeur et l'hydrolyse enzymatique ont été effectués selon le protocole décrit dans la demande de brevet français n° 85 06 027.

La paille de blé hachée a été trempée pendant 24 heures à température ambiante dans l'acide sulfurique 0,1 N. Après essorage, celle-ci a été traitée pendant 2 minutes à la pression de 14 bars (195°C) et pH 1,6 dans un appareil discontinu de prétraitement d'explosion à la vapeur.

L'hydrolyse enzymatique a été réalisée dans les conditions suivantes:
— concentration de substrat: 100 g (poids sec) par kg de suspension
— concentration de l'enzyme produite par *Trichoderma reesei*: 10 unités "papier filtre" par gramme de substrat
— pH: 4,8 — agent de neutralisation $Ca(OH)_2$
— température: 50°C; durée d'hydrolyse 24 heures

L'hydrolysat obtenu renferme: glucose 48,7 $g.1^{-1}$, xylose 27.2 $g.1^{-1}$, arabinose 2,9 $g.1^{-1}$, cellobiose 7,7 $g.1^{-1}$

2) Préparation du milieu de fermentation et stérilisation en présence de $Ca^{++}$

Dans un fermenteur de 6 litres de capacité, on introduit 3 litres d'hydrolysat, 1 litre d'eau de ville, 12 g (poids sec) de liqueur de macération de maïs, 12 g de sulfate d'ammonium et 5,2 g d'acide acétique. On neutralise ensuite le milieu jusqu'à pH 8 avec un lait de chaux (environ 6 g de CaO) et on stérilise le fermenteur à 110°C pendant 25 minutes. Après stérilisation, on maintient le fermenteur sous atmosphère d'azote stérile pendant toute la durée du refroidissement et on prélève stérilement dans un erlenmeyer de 350 ml de capacité, 200 ml de ce milieu (y compris la fraction insoluble) qui serviront pour la préculture.

3) Culture en fermenteur

On ensemence un tube de 10 ml de milieu RCM (Reinforced Clostridial Medium) avec 1 ml de suspension de la souche NCIB 6441 de *Clostridium acetobutylicum* dans son milieu de conservation. Après 17 heures d'incubation à 34°C en l'absence d'air la culture obtenue est transférée dans l'erlenmeyer précité contenant 200 ml d'hydrolysat traité à la chaux. Après 9 heures de croissance en anaérobiose, cette culture sert d'inoculum au fermenteur.

Au début de la fermentetion, le pH baisse du fait de l'excrétion des acides acétique et butyrique. Lorsqu'il atteint la valeur 5,5, il est maintenu constant par addition d'ammoniaque.

Après 60 heures de fermentation, on obtient 7,0 $g.1^{-1}$ d'acétone et 10,5 $g.1^{-1}$ de butanol contre 0,4 $g.1^{-1}$ d'acide acétique et 1,0 $g.1^{-1}$ d'acide butyrique. 100 litres de gaz ($H_2$ et $CO_2$) ont été produits.

Exemple 2: traitement avec $Mg^{++}$

1) Prétraitement et hydrolyse

L'hydrolysat est préparé dans les mêmes conditions que dans l'exemple 1 en remplaçant la chaux par la soude.

L'hydrolysat obtenu à la composition suivante: glucose 49,1 $g.1^{-1}$, xylose 30,1 $g.1^{-1}$, arabinose 3,7 $g.1^{-1}$, cellobiose 11,2 $g.1^{-1}$.

2) Préparation du milieu de fermentation et stérilisation en présence de $Mg^{++}$

On introduit dans le fermenteur 3 litres d'hydrolysat, 1 litre d'eau de ville et 12 g (poids sec) de liqueur de macération de maïs. Le milieu obtenu est neutralisé à pH 8,0 avec $NH_4OH$ puis additionné de 12 g de $MgCO^6$. Il est ensuite stérilisé à la température de 110°C pendant 25 minutes. Comme dans l'exemple 1, une partie aliquote de ce milieu est prélevée pour la préparation de l'erlenmeyer de préculture.

3) Culture en fermenteur

La chaîne d'ensemencement est identique à celle de l'exemple 1 et la fermentation est conduite sans régulation de pH.

Après 54 heures de fermentation, on obtient 7,9 $g.1^{-1}$ d'acétone, et 10,l $g.1^{-1}$ de butanol contre 0,25 $g.1^{-1}$ d'acide acétique et 1,15 $g.1^{-1}$ d'acide butyrique; 103 litres de gaz ont été produits.

Exemple 3: traitement avec $Al^{+++}$

1) Prétraitement et hydrolyse

L'hydrolysat utilisé est identique à celui de l'exemple 2.

2) Préparation du milieu de fermentation traité avec $Al^{+++}$ et stérilisation

On introduit dans le fermenteur 3 litres d'hydrolysat, 1 litre d'eau de ville, 12 g (poids sec) de liqueur de macération de maïs, 12 g de sulfate d'ammonium. Le milieu obtenu est neutralisé à pH 8,0 avec l'ammoniaque puis additionné de 12 g de sulfate d'aluminium. On stérilise et prépare le fermenteur comme dans l'exemple 1.

3) Culture en fermenteur

Elle est réalisée comme dans l'exemple 1.

Après 80 heures de fermentation, on obtient 6,5 $g.1^{-1}$ d'acétone et 9,8 $g.1^{-1}$ de butanol, contre 0,4 $g.1^{-1}$ d'acide acétique et 0,4 $g.1^{-1}$ d'acide butyrique. 84 l de gaz ont été produits.

Exemple 4: absence de traitement de l'invention (exemple comparatif)

On opère comme dans l'exemple 2 si ce n'est qu'on neutralise à l'ammoniaque à pH 8,0 sans addition de $MgCO_3$. Dans ces conditions aucune fermentation n'a lieu.

Exemple 5: absence de prétraitement (exemple comparatif)

On opère comms dans l'example 4, si ce n'est qu'on effectue l'hydrolyse enzymatique sur un lot de paille qui n'a pas subi de prétraitement. Le milieu obtenu n'est alors pas fermentescible.

Exemple 6:

On opère comme dans l'exemple 1, si ce n'est qu'on effectue l'hydrolyse enzymatique avec une préparation enzymatique commerciale (MAXA-ZYM de GIST BROCADES) qu'on utilise à la concentration de 10 unités "Papier filtre" par gramme de substrat.

L'hydrolysat obtenu renferme: glucose 42 $g.1^{-1}$, xylose 24 $g.1^{-1}$ arabinose 2,8 $g.1^{-1}$, cellobiose 9.0 $g.1^{-1}$.

Après de traitement par les ions $Ca^{++}$ et une fermentation d'une durée de 60 heures on obtient 6,0 $g.1^{-1}$ d'acétone et 9,7 $g.1^{-1}$ de butanol contre 0,8 $g.1^{-1}$ d'acide acétique, 1,5 $g.1^{-1}$ d'acide butyrique. 90 litres de gaz ont été produits.

Exemples 7 et 8: Séparation d'insoluble avant fermentation (exemples comparatifs)

On répète l'exemple 1; toutefois, après stérilisation, on centrifuge et on prélève seulement le liquide clair en vue de la fermentation. Le précipité est éliminé.

Quand on effectue la fermentation sur ce liquide clair, toutes choses égales par ailleurs, le rendement n'est que 4,6 $g.1^{-1}$ d'acétone et 7,3 $g.1^{-1}$ de butanol.

Le même expérience, effectuée cette fois sur le liquide clair provenant de la stérilisation de l'exemple 2, conduit aux rendements suivants: 5,1 $g.1^{-1}$ d'acétone et 8,1 $g.1^{-1}$ de butanol.

Ces exemples montrent donc l'importance de laisser dans le milieu, lors de la fermentation, au moins une fraction de l'insoluble formé avec la chaux.

**Revendications**

1. Procédé de production d'acétone et de butanol, dans lequel une matière lignocellulosique est soumise dans l'ordre, à un prétraitement par la vapeur d'eau et/ou par un acide, à une hydrolyse enzymatique par une cellulase et à une fermentation acétonobutylique du produit d'hydrolyse enzymatique, caractérisé en ce qu'après l'hydrolyse enzymatique et avant la fermentation le milieu est chauffé à au moins 50°C en présence d'ions calcium, magnésium et/ou aluminium, et en ce que le produit résultant de ce chauffage, y compris au moins une partie de la fraction insoluble, est soumis à la fermentation acétonobutylique.

2. Procédé selon la revendication 1, dans lequel le chauffage est inclus dans un traitement de stérilisation.

3. Procédé selon la revendication 1 ou 2, dans lequel le chauffage est réalisé à pH de 6,0 à 8,0.

4. Procédé selon l'une quelconque des revendications 1 à 3, dans lequel on opère en présence d'ions calcium et/ou magnésium.

5. Procédé selon la revendication 4, dans lequel les ions sont introduits sous forme d'hydroxyde ou de carbonate.

6. Procédé selon l'une des revendications 1 à 5, dans lequel le prétraitement est un traitement d'explosion par la vapeur d'eau sous pression à température d'au moins 150°C.

7. Procédé selon l'une des revendications 1 à 6, dans lequel la totalité de la fraction insoluble est maintenue dans le produit qui est soumis à la fermentation acétonobutylique.

## Patentansprüche

1. Verfahren zur Herstellung von Aceton und Butanol, bei dem ein Lignocellulosematerial nacheinander einer Vorbehandlung durch Wasserdampf und/oder eine Säure, einer enzymatischen Hydrolyse durch eine Cellulase und einer Acetonbutyl-Fermentation des enzymatischen Hydrolyseproduktes unterworfen wird, dadurch gekennzeichnet, daß nach der enzymatichen Hydrolyse und vor der Fermentation das Milieu auf mindestens 50°C in Gegenwart von Calcium-, Magnesium- und/oder Aluminiumionen erhitzt, und daß das bei diesem Erhitzen gebildete Produkt, das mindestens einen Teil der unlöslichen Fraktion enthält, der Acetonbutyl-Fermentation unterworfen wird.

2. Verfahren nach Anspruch 1, bei dem das Erhitzen in eine Sterilisationsbehandlung einbezogen wird.

3. Verfahren nach Anspruch 1 oder 2, bei dem das Erhitzen bei einem pH von 6,0 bis 8,0 durchgeführt wird.

4. Verfahren nach einem der Ansprüche 1 bis 3, bei dem man in Gegenwart von Calcium- und/ oder Magnesiumionen arbeitet.

5. Verfahren nach Anspruch 4, bei dem die Ionen in Form des Hydroxids oder Carbonats eingeführt werden.

6. Verfahren nach einem der Ansprüche 1 bis 5 bei dem die Vorbehandlung eine Explosionszerstäubungsbehandlung durch Wasserdampf unter Druck bei einer Temperatur von mindestens 150°C ist.

7. Verfahren nach einem der Ansprüche 1 bis 6, bei dem die Gesamtmenge der unlöslichen Fraktion in dem Produkt belassen wird, das der Acetonbutyl-Fermentation unterworfen wird.

## Claims

1. Process for the production of acetone and butanol, in which a lignocellulose substance successively undergoes a pretreatment by water vapour and/or an acid, an enzymatic hydrolysis by a cellulase and an acetonobutylic fermentation of the enzymatic hydrolysis product, characterized in that following the enzymatic hydrolysis and prior to fermentation the medium is heated to at least 50°C in the presence of calcium, magnesium and/or aluminium ions and in that the product resulting from said heating, including at least part of the insoluble fraction, is subject to acetonobutylic fermentation.

2. Process according to claim 1, wherein heating is included in a sterilization treatment.

3. Process according to claims 1 or 2, wherein heating is performed at a pH of 6 to 8.

4. Process according to any one of the claims 1 to 3, wherein working takes place in the presence of calcium and/or magnesium ions.

5. Process according to claim 4, wherein the ions are introduced in hydroxide or carbonate form.

6. Process according to any one of the claims 1 to 5, wherein the pretreatment is a pressurized water vapour explosion treatment at a temperature of at least 150°C.

7. Process according to one of the claims 1 to 6, wherein the entire insoluble fraction is kept in the product subject to acetonobutylic fermentation.